(19) Europäisches Patentamt
European Patent Office
Office européen des brevets

(11) Numéro de publication : **0 491 600 A1**

(12) **DEMANDE DE BREVET EUROPEEN**

(21) Numéro de dépôt : **91403363.4**

(22) Date de dépôt : **12.12.91**

(51) Int. Cl.$^5$ : **C07F 5/04,** C07C 69/92, C07C 309/73, C07C 43/23, C07C 205/38, C07F 7/18

(30) Priorité : **18.12.90 FR 9015810**

(43) Date de publication de la demande : **24.06.92 Bulletin 92/26**

(84) Etats contractants désignés : **BE CH DE FR GB IT LI LU NL**

(71) Demandeur : **ROUSSEL-UCLAF**
**35, Boulevard des Invalides**
**F-75007 Paris (FR)**

(72) Inventeur : **Brayer, Jean-Louis**
**42, rue Jules Dubrulle**
**F-60440 Nanteuil le Haudoin (FR)**
Inventeur : **Calvo, Daniel**
**128, Avenue Pierre Kerautret**
**F-93230 Romainville (FR)**
Inventeur : **Ottello, François**
**33, Allée Bossuet**
**F-93190 Livry Gargan (FR)**

(74) Mandataire : **Vieillefosse, Jean-Claude et al**
**Roussel-Uclaf 111, route de Noisy B.P. 9**
**F-93230 Romainville (FR)**

(54) **Nouveau procédé de monofontionnalisation régiospécifique d'un hydroxy phénolique sur un polyphénol.**

(57) L'invention concerne un procédé de préparation des produits (I) :

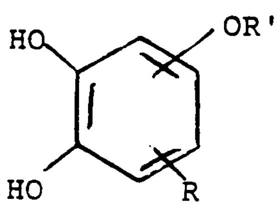

(I)

R étant hydrogène, alkyle, alcényle, cyano, formyle, carboxy, alcoxy carbonyle, [(arylalkyl) oxy] carbonyle ou halogène et R′ un groupe protecteur, caractérisé en ce que l'on traite un produit (II) :

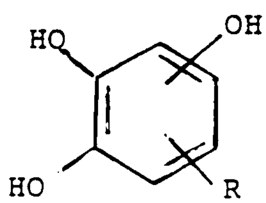

(II)

par une base, puis par un borate d'alkyle, pour obtenir le produit (III) :

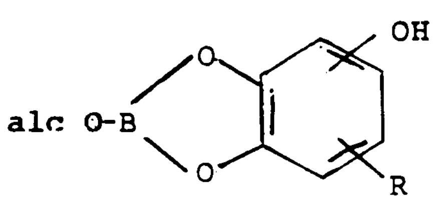

(III)

dont on protège l'hydroxy libre, puis que l'on hydrolyse.
Les produits (I) sont des intermédiaires utiles en synthèse organique.

EP 0 491 600 A1

La présente invention a pour objet un nouveau procédé de monofonctionnalisation régiospécifique d'un hydroxy phénolique sur un polyphénol.

L'invention a ainsi pour objet un procédé de préparation des produits de formule (I) :

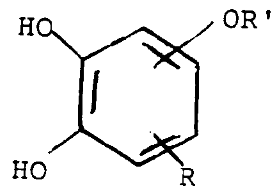

(I)

dans laquelle R représente un atome d'hydrogène, un radical alkyle, alcényle ou alcynyle, linéaire ou ramifié, renfermant au plus 8 atomes de carbone, un radical cyano, formyle, carboxy, alcoxycarbonyle renfermant au plus 9 atomes de carbone, [(arylalkyl) oxy] carbonyle renfermant au plus 12 atomes de carbone ou un atome d'halogène et R′ représente un groupe protecteur du radical hydroxy, caractérisé en ce que l'on traite un polyphénol de formule (II) :

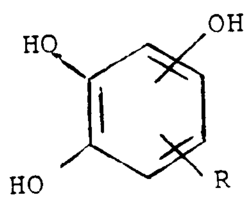

(II)

dans laquelle R est défini comme précédemment, par une base pour obtenir le dianion correspondant, que l'on traite par un borate de trialkyle de formule $B(Oalc)_3$, dans laquelle alc représente un radical alkyle renfermant de 1 à 6 atomes de carbone, pour obtenir un produit de formule (III) :

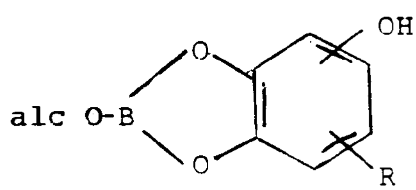

(III)

dans laquelle alc et R sont définis comme précédemment, que l'on traite par un réactif susceptible d'introduire un groupe protecteur sur le radical hydroxy, pour obtenir le produit correspondant comportant un radical hydroxy protégé, produit que l'on traite par un agent d'hydrolyse de la liaison oxygène - bore pour obtenir le produit de formule (I) attendu.

Lorsque R représente un radical alkyle, il peut être en particulier un radical méthyle, éthyle, propyle, butyle, pentyle ou hexyle, linéaire ou ramifié.

Lorsque R représente un radical alcényle, il peut être en particulier un radical vinyle, allyle, isopropényle, 2-méthyl allyle, isobutényle.

Lorsque R représente un radical alcynyle, il peut être en particulier un radical éthynyle, propargyle, butynyle ou pentynyle.

Lorsque R représente un radical alcoxycarbonyle, il peut être en particulier un radical méthoxycarbonyle, éthoxycarbonyle, propoxycarbonyle, isopropoxycarbonyle, butoxycarbonyle, isobutoxycarbonyle, ou tert-butoxycarbonyle.

Lorsque R représente un radical [(arylalkyl) oxy] carbonyle, il peut être en particulier un radical benzyloxy carbonyle.

Lorsque R représente un atome d'halogène, il peut être un atome de fluor, de chlore, de brome ou d'iode.

Le groupe protecteur du radical hydroxy peut être tout groupe connu de l'homme du métier dans la chimie des phénols et, en particulier, ceux cités dans l'ouvrage de référence "Protective Groups in Organic Synthesis" de Th. Green. (John Wiley et Sons Ed.). A titre purement d'exemples, on peut citer les radicaux alkyles primaires et secondaires, notamment méthyle, éthyle et isopropyle ; alcényles, notamment allyle ; [(alkyloxy) alkyloxy] alkyle, notamment [(méthoxy) éthoxy] méthyle ; alcanoyle, notamment pivaloyle ; aryle éventuellement substitué, notamment nitrophényle, arylalkyle, notamment benzyle ou phénéthyle ; aryl sulfonyle, notamment p-toluène sulfonyle ; aryl silyle ou alkyl aryl silyle, notamment diphényl tert-butyl silyle.

Dans des conditions préférentielles d'exécution du procédé de l'invention :

– on traite le produit de formule (II) par au moins deux équivalents d'une base, laquelle est de préférence l'hydrure de sodium, ou un organométallique compatible avec le groupe R, notamment un alkyl lithium ;
– la réaction de blocage par le borate est conduite au sein d'un solvant organique aprotique anhydre, notamment le tétrahydrofuranne ou le diméthylformamide ;
– le borate utilisé est un borate de triméthyle, triéthyle, tripropyle, triisopropyle ou tributyle ;
– on opère sans isolement des produits intermédiaires formés et à température ambiante ;
– le réactif d'introduction du groupe protecteur du radical hydroxy est un halogénure ;
– l'agent d'hydrolyse est un acide, notamment l'acide chlorhydrique, ou un oxydant, notamment l'eau oxygénée.

La structure indiquée plus haut du borate cyclique intermédiaire de formule (III) ne préjuge en rien du mécanisme de la réaction de blocage par le borate de trialkyle. Il est toutefois vraisemblable que cet intermédiaire (III) donne naissance in situ, par des réactions équilibrées, à un certain nombre d'autres intermédiaires, dont le boronate de formule (IV) :

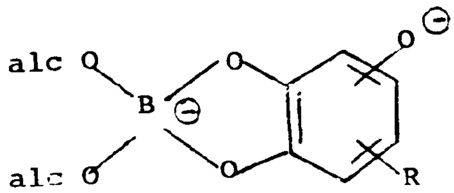

(IV)

A titre purement indicatif, on peut en déduire que la structure du produit intermédiaire comportant un radical hydroxy protégé, résulte de celle du boronate ci-dessus et est donc vraisemblablement la suivante :

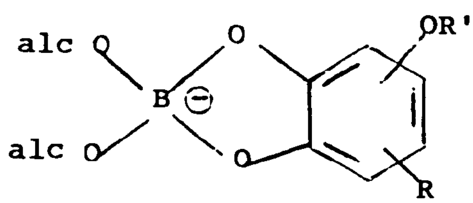

5V)

Des procédés de préparation de produits de type (I) ont déjà été décrits dans le passé, dont certains utilisaient un dérivé du bore. C'est notamment le cas du procédé décrit par Scheline (Acta. Chem. Scand. 20 (1966) n° 4 p. 1182) et repris par Kato (Synth. Commun. (1980) - p. 172). Ce procédé qui réalise le blocage de deux hydroxy 1,2, utilise le borate de sodium. On peut rappeler également que, plus généralement, des blocages de diols par des dérivés de l'acide borique ont été décrits, notamment dans la chimie des sucres. Ces blocages utilisent en particulier des dérivés de l'acide borique de formule $A\text{-}B(OH)_2$, dans laquelle A représente un radical aryle ou polystyryle (Fréchet et coll. J. Am. Chem. Soc. 101 (1979) p. 432).

Le procédé de la présente invention présente d'importants avantages par rapport à ceux décrits dans la littérature. Il utilise des réactifs commerciaux, peu onéreux et très faciles à manipuler, il ne nécessite l'isolement d'aucun intermédiaire et procure d'excellents rendements, et surtout, il est très général, du fait qu'il évite l'utilisation de conditions à la fois basiques et aqueuses.

L'utilisation de telles conditions limite les procédés de l'art antérieur à la préparation de composés de type (I) dans lesquels R′ représente un radical alkyle inférieur et pratiquement méthyle. L'utilisation de conditions aqueuses dans ces procédés n'autorise pas la mise en oeuvre de réactifs de protection instables ou réagissant trop lentement sur le phénol dans ces conditions ; on ne permet pas la préparation de produits dont l'hydroxy protégé est instable dans ces conditions.

Le procédé de la présente invention offre en outre l'avantage de préserver l'intégrité du groupe R, ce qui n'est pas le cas des procédés de l'art antérieur.

Les produits de formule (I) sont des produits très utiles en synthèse organique, notamment en raison de la présence du groupe R, à partir duquel des réactions d'homologations en particulier, sont possibles. Toutefois, des substitutions sur le cycle sont bien entendu, également possibles. Parmi les applications décrites, on peut citer les synthèses d'alcaloïdes du type cotarnine et, plus généralement, les synthèses utilisant au départ l'acide

gallique, des analogues ou des dérivés (voir par exemple les références G.J. Kapadia et coll. J. Pharm. Sci. 58 n° 9 (1969) p. 1157, ou encore Y. Takuma et al. Mitsubishi Chemical. "An efficient synthesis of cotamine" Congrès IUPAC de Kyoto 29/05-03/06/1988 ou encore Y. Kato. Synth. Comm. 172 (1980).

Les exemples suivants illustrent l'invention sans toutefois la limiter

**Exemple 1 : 3-[(4-méthyl phényl) sulfonyloxy] 4,5-dihydroxy benzoate de méthyle.**

On introduit, sous gaz inerte, 1 g de gallate de méthyle dans 30 cm3 de diméthylformamide anhydre, ajoute lentement 0,492 g d'hydrure de sodium en suspension à 53 % dans l'huile et maintient sous agitation à température ambiante pendant une heure. On ajoute alors 9,953 $cm^3$ de borate de triéthyle, poursuit l'agitation pendant 30 minutes et introduit en 2 heures à température ambiante une solution de 1,036 g de chlorure de tosyle dans 10 $cm^3$ de diméthylformamide anhydre. On agite pendant 2 heures à température ambiante puis, après un repos de 15 heures, verse dans un mélange d'eau glacée et d'acide chlorhydrique N (pH$\simeq$ 3). On essore les cristaux, les rince à l'eau et les sèche sous pression réduite. On obtient 1,78 g de produit attendu brut, que l'on lave à l'éther isopropylique. On obtient finalement 1,66 g du produit attendu (Rdt : 90,4 %).

Spectre RMN -($CDCl_3$ - 60 MHz)
H du hydroxy : 3 ppm
H aromatiques de 7,05 à 7,9 ppm
H du méthyl de l'ester : 3,86 ppm
H du méthyl en 4 du phényl : 2,5 ppm

**Exemple 2 : 3-[(4-méthyl phényl) sulfonyloxy] 4,5-dihydroxy benzoate de méthyle.**

On place sous gaz inerte 1 l de tétrahydrofuranne anhydre puis y ajoute sous agitation, à température ambiante, 118 g d'hydrure de sodium en suspension dans l'huile. On ajoute ensuite en 15 minutes 555 $cm^3$ de borate de triéthyle, puis en 2 heures une solution de 379 g de gallate de méthyle dans 1,8 l de tétrahydrofuranne. On maintient sous agitation pendant 15 heures, puis ajoute 283 g de carbonate de potassium anhydre. On ajoute ensuite en 30 minutes une solution de 410 g de chlorure de tosyle dans 820 cm3 de tétrahydrofuranne et maintient sous agitation pendant 24 heures, puis 2 heures au reflux, on refroidit et verse sur un mélange glace - acide chlorhydrique 2 N (2 litres) et extrait à l'acétate d'éthyle. On lave la phase organique à l'eau saturée de chlorure de sodium, sèche et évapore le solvant. On empâte le résidu dans l'éther isopropylique, puis dans le chlorure de méthylène, essore, sèche et obtient 576 g de produit attendu [(Rdt : 89,8 %), (F = 196°C)], identique à celui obtenu à l'exemple 1.

**Exemple 3 : 3-[(4-méthyl phényl) sulfonyloxy] 4,5-dihydroxy benzoate de propyle.**

On place sous gaz inerte 400 $cm^3$ de tétrahydrofuranne anhydre et y ajoute 62 g d'hydrure de sodium en suspension dans l'huile. On ajoute ensuite en 5 minutes, sous agitation, 294 $cm^3$ de borate de thiéthyle puis, en 2h45 minutes, à + 15°C, une solution de 230 g de gallate de propyle dans 600 $cm^3$ de tétrahydrofuranne. On maintient ensuite sous agitation, à + 18°C pendant 2 heures, puis ajoute une solution de 217 g de chlorure de tosyle dans 500 $cm^3$ de tétrahydrofuranne, puis 70 g de carbonate de potassium et maintient sous agitation pendant 17 heures à température ambiante.

On porte ensuite au reflux et rajoute de nouveau 70 g de carbonate de potassium, puis refroidit et verse sur un mélange de glace et de 400 $cm^3$ d'acide chlorhydrique 22°Bé. On sature en chlorure de sodium, extrait à l'acétate d'éthyle, sèche la phase organique, évapore le solvant, empâte le résidu au chlorure de méthylène et le sèche. On obtient 324,6 g de produit attendu F = 152°C (Rdt : 81,7 %).

**Exemple 4 : 3-(benzyloxy) 4,5-dihydroxy benzoate de méthyle.**

On mélange sous gaz inerte 1 g de gallate de méthyle et 30 $cm^3$ de diméthylformamide anhydre, puis ajoute à 15°C, 0,49 g d'hydrure de sodium en suspension à 53 % dans l'huile. Après 1 heure, on ajoute à température ambiante 0,606 $cm^3$ de borate de triméthyle puis, en 1 heure, une solution de 0,645 $cm^3$ de bromure de benzyle dans 10 $cm^3$ de diméthylformamide. On maintient sous agitation à température ambiante pendant 3 heures puis verse dans un mélange d'eau de glace et de 10 $cm^3$ d'acide chlorhydrique 2N. On extrait à l'acétate d'éthyle, sèche la phase organique et l'amène à sec. On reprend le résidu dans un mélange éther éthylique - éther isopropylique, filtre les cristaux et obtient, après séchage, 1,47 g de produit attendu. On recristallise 1,26 g du produit obtenu dans le toluène. On obtient finalement 1,02 g de produit pur (Rdt : 80,2 %).

<u>Analyse</u> : $C_{15}H_{14}O_5$ : 274,27

| | | |
|---|---|---|
| Calculé : | C % 65,7 | H % 5,1 |
| Trouvé | 65,4 | 5,1 |

<u>Spectre RMN</u> : $CDCl_3$ 60MHz

| | |
|---|---|
| H du méthyle de l'ester | : 3,9 ppm |
| H du $CH_2$ du benzyle | : 5,17 ppm |
| H aromatiques | : 7,32 à 7,45 ppm. |

**<u>Exemple 5</u> : 3-(benzyloxy) 4,5-dihydroxy benzoate de méthyle.**

On opère comme indiqué à l'exemple 4 en utilisant différents borates figurant dans le tableau ci-dessous. On a obtenu dans tous les cas le produit attendu pur, avec le rendement indiqué.

| Borate utilisé | Rendement obtenu |
|---|---|
| Borate de tripropyle | 81% |
| Borate de triisopropyle | 97% |
| Borate de tributyle | 81% |

**<u>Exemple 6</u> : 3-(allyloxy) 4,5-dihydroxy benzoate de méthyle**

On mélange sous gaz inerte 5 g de gallate de méthyle et 150 cm³ de diméthylformamide, puis ajoute en 10 mn à + 15°C, 2,46 g d'hydrure de sodium en suspension à 53 % dans l'huile. Après 1 heure, on ajoute lentement 4,76 cm³ de borate de triéthyle puis, en 1 heure, 2,35 cm³ de bromure d'allyle dans 50 cm³ de diméthylformamide. On maintient sous agitation pendant 2 heures à température ambiante puis verse dans un mélange eau - glace - acide chlorhydrique. On extrait à l'acétate d'éthyle, lave à l'eau, sèche et évapore à sec. On chromatographie le résidu sur silice en éluant au mélange cyclohexane - acétate d'éthyle (6 - 4) et obtient 5,2 g d produit attendu. F = 109°C.

<u>Analyse</u> : $C_{11}H_{12}O_5$ = 224,216

| | | |
|---|---|---|
| Calculé : | C % 58,93 | H % 5,4 |
| Trouvé : | 59,2 | 5,5 |

<u>Spectre RMN</u> : $CDCl_3$ 60 MHz

| | |
|---|---|
| H du $CH_3$ de l'ester | : 3,92 ppm |
| H du $CH_2$ de l'allyle | : 4,63 - 4,72 ppm |
| H du $-CH=CH_2$ | : 5,25 - 6,46 ppm |
| H des 2 hydroxy | : 5,75 - 6,10 ppm |
| H aromatiques | : 7,25 à 7,44 ppm |

**<u>Exemple 7</u> : 3-(isopropyloxy) 4,5-dihydroxy benzoate de méthyle.**

On opère comme indiqué à l'exemple 6, en utilisant au départ 1 g de gallate de méthyle, 0,96 $cm_3$ de borate de triéthyle et 0,56 $cm_3$ de bromure d'isopropyle. On ajoute à nouveau 0,56 cm³ de bromure d'isopropyle, et

chauffe alors à 55°C pendant 8 h. On verse sur le mélange eau-glace-acide chlorhydrique 2N, extrait à l'acétate d'éthyle et chromatographie le produit sur silice comme à l'exemple 6. On obtient 0,8 g de produit attendu (Rdt : 65 %).

| | | | |
|---|---|---|---|
| Analyse : | $C_{11}H_{14}O_5$ : 226,232 | | |
| Calculé : | C % 58,4 | H % | 6,24 |
| Trouvé : | 58,5 | | 6,3 |

Spectre RMN - $CDCl_3$ - 60 MHz

| | |
|---|---|
| H des méthyles de l'isopropyle | : 1,32 - 1,42 ppm |
| H du CH de l'isopropyle | : 4,37 à 4,97 ppm |
| H du méthyle de l'ester | : 3,9 ppm |
| H aromatiques | : 7,23 à 7,37 ppm |
| H des 2 OH | : 5,63 à 6,05 ppm |

**Exemple 8 : 3-[(méthoxy éthoxy) méthoxy] 4,5-dihydroxy benzoate de méthyle.**

On opère comme indiqué à l'exemple 6, en utilisant au départ 1 g de gallate de méthyle, 0,96 $cm^3$ de borate de triéthyle et 0,682 $cm^3$ de chlorure de (méthoxy éthoxy) méthyle.

On obtient après chromatographie dans les conditions de l'exemple 6, 0,927 g de produit attendu, sous forme d'une huile (Rdt : 63 %)

| | | | |
|---|---|---|---|
| Analyse : | $C_{12}H_{16}O_7$ : 272,26 | | |
| Calculé : | C % 52,94 | H % | 5,92 |
| Trouvé : | 53,0 | | 6,3 |

Spectre RMN : $CDCl_3$ - 60 MHz

| | |
|---|---|
| H de $O\text{-}CH_3$ | : 3,45 ppm |
| H du $CH_3$ de l'ester | : 3,88 ppm |
| H de $O\text{-}CH_2\text{-}CH_2\text{-}O$ | : 3,5 à 4 ppm |
| H de $O\text{-}CH_2\text{-}O$ | : 5,27 ppm |
| H aromatiques | : 7,35 à 7,45 ppm |

**Exemple 9 : 3-(diphényl tert-butyl silyloxy) 4,5-dihydroxy benzoate de méthyle.**

On opère comme indiqué à l'exemple 6, en utilisant au départ 1 g de gallate de méthyle, 0,96 $cm^3$ de borate de triéthyle et 1,482 $cm^3$ de chlorure de diphényl tert-butyl silyle. On obtient après chromatographie dans les conditions de l'exemple 6, 1,63 g de produit attendu, se présentant sous forme d'une huile (Rdt : 71 %).

| | | | |
|---|---|---|---|
| Analyse : | $C_{24}H_{26}O_5Si$ : 422,56 | | |
| Calculé : | C % 68,2 | H % | 6,2 |
| Trouvé : | 69,2 | | 6,4 |

Spectre RMN : $CDCl_3$ - 60 MHz

| | |
|---|---|
| H de tBuSi | : 1,13 ppm |
| H de $CH_3$ ester | : 3,63 ppm |
| H des 2 OH | : 5,47 ppm |
| H aromatiques | : 6,75 à 78,75 ppm. |

**Exemple 10 : 3-(pivaloyloxy) 4,5-dihydroxy benzoate de méthyle.**

On opère comme indiqué à l'exemple 6, en utilisant au départ 1 g de gallate de méthyle, 0,96 $cm^3$ de borate de triéthyle et 0,66 $cm^3$ de chlorure de pivaloyle. On obtient après chromatographie sur silice en éluant au mélange cyclohexane-acétate d'éthyle (6-4) à 1‰ de triéthylamine, 1,07 g de produit attendu sous forme d'une huile
(Rdt : 73,5 %)

| | | |
|---|---|---|
| Analyse : | $C_{13}H_{16}O_6$ | |
| Calculé : | C % 58,2 | H % 6,0 |
| Trouvé : | 58,1 | 5,9 |

Spectre RMN : $CDCl_3$ 60MHz
H de tBu : 1,4 ppm
H de $CH_3$ester : 3,87 ppm
H aromatiques : 7,33 à 7,36 ppm
H des OH : 6,3 ppm.

**Exemple 11 : 3-[(2-nitro phényl) oxy] 4,5-dihydroxy benzoate de méthyle.**

On opère comme indiqué à l'exemple 6, en utilisant au départ 5,525 g de gallate de méthyle, 2,72 g d'hydrure de sodium, 5,8 $cm^3$ de borate de triéthyle et 3,5 $cm^3$ d'2-nitro fluorobenzène. On obtient après chromatographie du produit brut sur silice en éluant au mélange cyclohexane - acétate d'éthyle (1-1), 7,9 g de produit attendu cristallisé (Rdt : 86,3 %)

| | | | |
|---|---|---|---|
| Analyse : | $C_{14}H_{10}NO_7$ | 305,25 | |
| Calculé : | C % 55,09 | H % 3,63 | N % 4,6 |
| Trouvé : | 55,3 | 3,6 | 4,6 |

Spectre RMN (DM50 - 250 MHz)
H de l'ester : 3,78 ppm
H aromatiques : 713 et 7,38 pm, ainsi que 6,91 - 7,61 - 7,26 et 8,63 ppm.

**Exemple 12 : 1-(allyloxy) 2,3-dihydroxy benzène.**

On agite pendant 3 heures sous gaz inerte, un mélange de 3,8 g de pyrogallol, 76 $cm^3$ de diméthylformamide anhydre et 2,72 g d'hydrure de sodium en suspension à 53 % dans l'huile, puis ajoute 5,27 $cm^3$ de borate de triéthyle, agite pendant 30 minutes, ajoute en 45 minutes 2,6 $cm^3$ de bromure d'allyle en solution dans 26 $cm^3$ de diméthylformamide et agite pendant 16 heures. On verse dans un mélange eau-glace-acide chlorhydrique N, extrait à l'acétate d'éthyle et chromatographie le produit brut sur silice, en éluant au mélange cyclohexane-acétate d'éthyle 7-3. On obtient 3,8 g de produit attendu se présentant sous forme de cristaux blancs (Rdt : 76,3 %).
Spectre RMN $CDCl_3$ 60 MHz
H du $-CH_2-$ de l'allyle : 4,58 ppm
H du $=CH_2$ de l'allyle : 5,35 ppm
H du -CH = de l'allyle : 6,06 ppm
H aromatiques : 6,47 à 6,73 ppm
H des 2 OH : 5,53 ppm.

**Exemple 13 : 1-[(2-nitro phényl) oxy] 2,3-dihydroxy benzène.**

On mélange sous gaz inerte 18,9 g de pyrogallol, 380 $cm^3$ de diméthylformamide anhydre et 13,6 g d'hydrure de sodium en suspension à 53 % dans l'huile. On agite 45 minutes à température ambiante, puis ajoute 29 $cm^3$ de borate de triéthyle, agite de nouveau pendant 30 minutes, puis ajoute en 1 heure à 0°C, 17,6 $cm^3$ d'2-nitro fluorobenzène et agite pendant 20 heures à température ambiante. On verse dans un mélange eau-glace - acide chlorhydrique N, essore le précipité, le lave à l'éther de pétrole, le reprend à l'éther éthylique, lave à l'acide chlorhydrique N et obtient environ 27 g de produit attendu (Rdt ~ 76 %) se présentant sous la forme de cristaux beiges.
Spectre RMN (DM50 - 250MHz)
H aromatiques : 6,46 - 6,55 - 6,67 à 6,74 - 6,81 - 6,91, 7,16 et 7,53 ppm.

**Exemple 14 : 1-[(4-méthyl phényl) sulfonyloxy] 2,3-dihydroxy benzène.**

On opère comme indiqué à l'exemple 13, en utilisant au départ 18,9 g de pyrogallol 26,3 $cm^3$ de borate de triéthyle et 28,6 g de chlorure de tosyle. On obtient 32 g de produit brut se présentant sous forme d'une résine rouge (Rdt : 76,2 %).

Spectre IR ($CHCl_3$)
Absorptions à 3547 $cm^{-1}$ (OH phénoliques)
" 1598 - 1499 - 1482 $cm^{-1}$ (aromatiques)
" 1365 - 1192 - 1180 - 1166 $cm^{-1}$ ($SO_2$)

**Exemple 15 : 1-(benzyloxy) 2,3-dihydroxy benzène.**

On opère comme indiqué à l'exemple 13, en utilisant au départ 25,22 g de pyrogallol, 35,1 $cm^3$ de borate de triéthyle et 23,76 $cm^3$ de bromure de benzyle. On obtient après chromatographie sur silice en éluant au mélange cyclohexane - acétate d'éthyle (6-4), 33,8 g de produit attendu se présentant sous forme d'une huile épaisse rouge (Rdt : 78 %).
Spectre RMN $CDCl_3$ 60 MHz

| | |
|---|---|
| H du $CH_2$ benzylique | : 5,13 ppm |
| H aromatiques | : 6,5 à 7,37 ppm |
| H des 2 OH | : 6,63 ppm |

**Exemple 16 : 1-[(4-méthyl phényl) sulfonyloxy 3,4-dihydroxy benzène.**

On mélange sous gaz inerte 1,37 g de 1,2,4-trihydroxy benzène et 40 $cm^3$ de diméthylformamide anhydre, puis ajoute à 15,20°C, lentement, 0,984 g d'hydrure de sodium en suspension à 53 % dans l'huile. On agite 1 heure puis ajoute 1,906 $cm^3$ de borate de triéthyle, agite de nouveau 1 heure, puis ajoute en 2 heures une solution de 2,072 g de chlorure de tosyle dans 15 $cm^3$ de diméthylformamide et agite pendant 4 heures à température ambiante. On verse sur un mélange eau-glace acide chlorhydrique, extrait à l'acétate d'éthyle, évapore à sec et lave le résidu brut à l'éther de pétrole. On chromatographie sur silice en éluant au mélange cyclohexane-acétate d'éthyle (1.1) et obtient 1,53 g de produit attendu que l'on cristallise dans un mélange flugène-éther isopropylique.
Spectre RMN : $CDCl_3$ 60 MHz

| | |
|---|---|
| H du $CH_3$ en 4 du phényl : | 2,47 ppm |
| H aromatiques : | 6,28 à 7,88 ppm |
| H des 2 OH : | 6,33 ppm |

**Exemple 17 : 1-(benzyloxy) 3,4-dihydroxy benzène.**

On mélange sous gaz inerte, 1,37 g de 1,2,4-trihydroxy benzène et 30 $cm^3$ de diméthylformamide anhydre puis ajoute à 15,20°C, 0,984 g d'hydrure de sodium en suspension à 53 % dans l'huile. On agite pendant 1 heure , puis on ajoute 1,906 $cm^3$ de borate de trièthyle. On agite de nouveau pendant 1 heure, puis ajoute en

2 heures, 1,29 $cm^3$ de bromure de benzyle en solution dans 20 $cm^3$ de diméthylformamide. On maintient sous agitation pendant 16 heures à température ambiante, puis verse sur un mélange eau-glace acide chlorhydrique N, extrait à l'acétate d'éthyle et évapore à sec. On lave l'extrait à l'éther de pétrole cyclohexane-acétate d'éthyle (6-4). On obtient 1,36 g de produit attendu cristallisé (Rdt : 58 %)

Spectre RMN : $CDCl_3$ 60 MHz

| | |
|---|---|
| H des 20 H | : 4,57 ppm |
| H du $CH_2$ benzylique | : 5,03 ppm |
| H aromatiques | : 6,37 à 7,43 ppm. |

## Revendications

**1)** Procédé de préparation des produits de formule (I) :

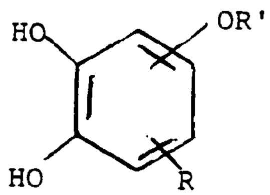

(I)

dans laquelle R représente un atome d'hydrogène, un radical alkyle, alcényle ou alcynyle, linéaire ou ramifié, renfermant au plus 8 atomes de carbone, un radical cyano, formyle, carboxy, alcoxycarbonyle renfermant au plus 9 atomes de carbone, [(arylalkyl) oxy] carbonyle renfermant au plus 12 atomes de carbone ou un atome d'halogène et R′ représente un groupe protecteur du radical hydroxy, caractérisé en ce que l'on traite un polyphénole de formule (II) :

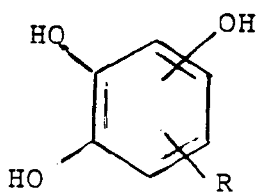

(II)

dans laquelle R est défini comme précédemment, par une base pour obtenir le dianion correspondant, que l'on traite par un borate de trialkyle de formule B $(Oalc)_3$, dans laquelle alc représente un radical alkyle renfermant de 1 à 6 atomes de carbone, pour obtenir un produit de formule (III) :

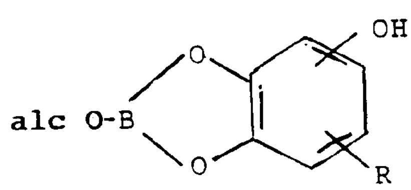

(III)

dans laquelle alc et R sont définis comme précédemment, que l'on traite par un réactif susceptible d'introduire un groupe protecteur sur le radical hydroxy, pour obtenir le produit correspondant sur le radical hydroxy protégé, produit que l'on traite par un agent d'hydrolyse de la liaison oxygène - bore pour obtenir le produit de formule (I) attendu.

**2)** Procédé selon la revendication 1, caractérisé en ce que l'on traite le produit de formule (II) par au moins deux équivalents d'une base qui est l'hydrure de sodium ou un alkyl lithium.

**3)** Procédé selon la revendication 2, caractérisé en ce que la base est l'hydrure de sodium.

**4)** Procédé selon l'une quelconque des revendications 1 à 3, caractérisé en ce que l'on opère le blocage par le borate de trialkyle au sein d'un solvant organique aprotique.

**5)** Procédé selon la revendication 4, caractérisé en ce que l'on opère au sein de tétrahydrofuranne ou du

diméthylformamide.

**6)** Procédé selon l'une quelconque des revendications 1 à 5, caractérisé en ce que l'on utilise un borate de triméthyle, triéthyle, tripropyle, triisopropyle ou tributyle.

**7)** Procédé selon l'une quelconque de revendications 1 à 6, caractérisé en ce qu'il est effectué sans isolement des produits intermédiaires formés et à température ambiante.

**8)** Procédé selon l'une quelconque des revendications 1 à 7, caractérisé en ce que l'agent d'hydrolyse est un acide ou un oxydant.

**9)** Procédé selon la revendication 8, caractérisé en ce que l'agent d'hydrolyse est l'acide chlorhydrique.

Office européen des brevets

# RAPPORT DE RECHERCHE EUROPEENNE

Numero de la demande

EP 91 40 3363

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | FR-A-1 566 626 (DOW CORNING)<br>* résumé; tableau II *<br>--- | 1-9 | C07F 5/04<br>C07C69/92<br>C07C309/73<br>C07C43/23<br>C07C205/38<br>C07F7/18 |
| D,A | ACTA CHEMICA SCANDINAVICA<br>vol. 20, no. 4, 1966,<br>page 1182;<br>R. R. SCHELINE: 'A RAPID SYNTHESIS OF 3-O-METHYLGALLIC ACID'<br>* le document en entier *<br>--- | 1-9 | |
| A | CHEMICAL ABSTRACTS, vol. 46, no. 9,<br>10 Mai 1952, Columbus, Ohio, US;<br>M. SHIMIZU ET AL: 'SOLUBILIZATION OF FLAVONOIDS II. PARTIAL METHYLATION OF RUTIN AND QUERCETIN WITH DIAZOMETHANE'<br>colonne 4004B ;<br>* abrégé *<br>--- | 1-9 | |
| A | CHEMICAL ABSTRACTS, vol. 46, no. 9,<br>10 Mai 1952, Columbus, Ohio, US;<br>M. SHIMIZU: 'SOLUBILIZATION OF FLAVONOIDS III. SOLUBILIZATION MECHANISM'<br>colonne 4004C ;<br>* abrégé *<br>--- | 1-9 | **DOMAINES TECHNIQUES RECHERCHES (Int. Cl.5 )** |
| A | CHEMICAL ABSTRACTS, vol. 91, no. 15,<br>8 Octobre 1979, Columbus, Ohio, US;<br>abstract no. 123715Y,<br>S. M. TRIPATHI ET AL: 'BORON DERIVATIVES OF BENZALDIMINES AND SALICYLALDIMINES'<br>page 608 ;<br>* abrégé *<br>----- | 1-9 | C07F<br>C07C |

Le présent rapport a été établi pour toutes les revendications

| Lieu de la recherche | Date d'achèvement de la recherche | Examinateur |
|---|---|---|
| LA HAYE | 26 MARS 1992 | WRIGHT M.W. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)